(19) Europäisches Patentamt
European Patent Office
Office européen des brevets

(11) Publication number: 0 228 893 A2

(12) EUROPEAN PATENT APPLICATION

(21) Application number: 86310047.5

(22) Date of filing: 22.12.86

(51) Int. Cl.⁴: A 61 K 31/445
A 61 K 31/47

(30) Priority: 20.12.85 US 811799
17.01.86 US 819701

(43) Date of publication of application:
15.07.87 Bulletin 87/29

(84) Designated Contracting States:
AT BE CH DE ES FR GB GR IT LI LU NL SE

(71) Applicant: A.H. ROBINS COMPANY, INCORPORATED
1407 Cummings Drive P.O. Box 26609
Richmond Virginia 23261-6609(US)

(72) Inventor: Yanni, John Michael
4633 Newby's Bridge Road
Chesterfield Virginia 23832(US)

(72) Inventor: Walsh, David Allan
9504 Lakewater Court
Richmond Virginia 23229(US)

(74) Representative: Sheard, Andrew Gregory et al,
Kilburn & Strode 30, John Street
London WC1N 2DD(GB)

(54) **Arylalkyl-heterocyclic amines, n-substituted by aryloxyalkyl group in allergy treatment.**

(57) Allergy can be treated with substituted heterocyclic amines, wherein the active agents are expressed generally by the formula:

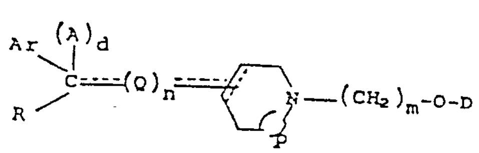

wherein p is zero, one or two; m is one to six inclusive; A is selected from hydrogen, hydroxy or cyano; d is zero or one; Q is $-CH-$, $-CH_2-$, or

OH
|
-C-;
|
H

n is zero or one and when Q is $-CH-$ and n is one, a double bond is formed with one of the adjacent carbons but not both at the same time, and when n and d are zero at the same time, a double bond is formed between the α carbon and a carbon of the central heterocyclic amine ring; Ar, D and R are selected from phenyl, substituted phenyl, pyridinyl, thienyl, furanyl or naphthyl and in addition, R may have the values benzyl, substituted benzyl, cycloalkyl or loweralkyl and D may additionally have the values; 2H-1-benzopyran-2-one, 4-oxo-4H-1-benzopyran-2-carboxylic acid loweralkyl ester, 2,3-dihydro-4H-1-benzopyran-4-one or 1,4-benzodioxan-loweralkyl-2-yl, and the pharmaceutically acceptable salts thereof.

EP 0 228 893 A2

# ARYLALKYL-HETEROCYCLIC AMINES, N-SUBSTITUTED BY ARYLOXYALKYL GROUP IN ALLERGY TREATMENT

0228893

This invention relates to the use, in the preparation of an anti-allergy agent, of piperidinyl, pyrrolidinyl, and homopiperidinyl derivatives substituted on nitrogen by aryloxyalkyl radicals and otherwise substituted by aryl (or diaryl)-alkanol, aryl, (or diaryl)-alkyl and aryl (or diaryl)alkylidine radicals. The invention thus contemplates the use of the aforementioned heterocyclic amine derivatives defined by Formula I hereinbelow in treating allergic phenomena which includes but is not limited to asthma, rhinitis, atopic dermatitis, chronic hives and the like.

Various systemic anti-allergy agents have long been known prior to this invention including, among others, aminophylline, theophylline, cortisosteroids, the disodium salt of 1,3-bis(2-carboxychromon-5-yloxy)-2-hydroxypropane, α-[(tertbutylamino)methyl]-3,5-dihydroxybenzylalcohol sulphate and oxatomide. The efficacy of some has suffered from undesirable side effects while others which are effective prophylactically are ineffective in acute manifestations of the allergic attack. By way of comparison, for example, the preferred compounds of the present invention are many times more potent than aminophylline and several times more potent than oxatomide.

Olefinic-4-substituted piperidino derivatives useful as antihistaminic, antiallergy agents and bronchodilators represented by the formula

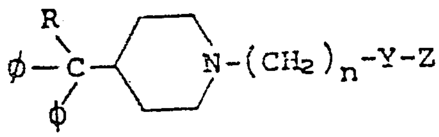

are disclosed in U. S. Patent 3,862,173 wherein R represents hydrogen or forms a double bond, Y represents -CH=CH- and Z represents thienyl, phenyl or phenyl substituted by halogen, alkyl, loweralkoxy, diloweralkylamino, pyrrolidino, piperidino, morpholino or N-loweralkylpiperazino. Compounds useful in the present invention differ in that an ether linkage is present and there is no unsaturation in the alkyl chain.

Diphenylmethylene-piperidineacetic acid derivatives useful as anti-allergic, anti-histaminic, and broncholytic agents are disclosed in European Patent 48705B and have the formula:

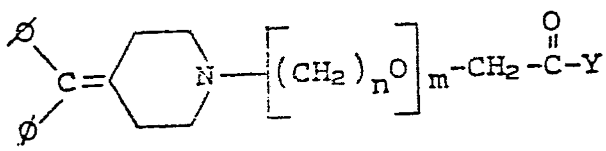

wherein Y is OH or $NR_1R_2$; compounds useful in the present invention have an aryl group next to the ether oxygen.

U. S. Patent 3,806,526 discloses 1-aroylalkyl-4-diphenylmethylpiperidines having antihistaminic, anti-allergenic and bronchodilator activity. In contrast, the compounds useful in the present invention have aryloxyalkyl radical on piperidine and pyrrolidine nitrogen rather than an aroylalkyl radical.

A number of the compounds useful in the present invention have been disclosed specifically and under generic formulas in U. S. Patents 3,922,276 and 3,956,296 as being useful as anti-inflammatory agents, tranquilizers and sedatives. These activities are not suggestive of use in treating allergy.

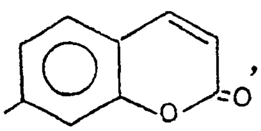, 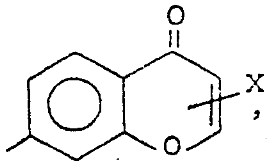, 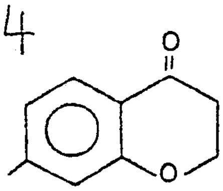, 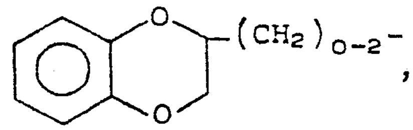,

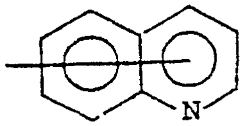,

or $Ar(CH_2)_{1-4}$-; X, Y, and Z are selected from the group consisting of hydrogen, loweralkyl, halogen, $-NO_2$, $-O-R^1$, $-\underset{\overset{\|}{O}}{C}-R^1$, $-CF_3$, $-C \equiv N$, $-\overset{\overset{O}{\|}}{C}-N(R^1)_2$, $-N(R^1)_2$, $-C(O)OR^1$, $-SO_2R^2$, $-SR^2$, $-S(O)R^2$, $-\underset{R^1}{\underset{|}{N}}-\underset{\overset{\|}{O}}{C}-R^1$, $-CH_2COOM$, $-SO_2N\begin{smallmatrix}R^1\\R^1\end{smallmatrix}$, $-\underset{R^1}{\underset{|}{N}}SO_2CH_3$, $-\underset{R^1}{\underset{|}{N}}\ \underset{\overset{\|}{O}}{C}-N\begin{smallmatrix}R^1\\R^1\end{smallmatrix}$, or $-\underset{R^1}{\underset{|}{N}}\ \underset{\overset{\|}{O}}{C}-OR^2$; $R^1$ is selected from hydrogen, loweralkyl, phenyl and phenylloweralkyl; $R^2$ is selected from loweralkyl, phenyl and phenylloweralkyl; M is a pharmaceutically acceptable metal ion; and the pharmaceutically acceptable salts thereof, including acid addition salts, quaternary salts and hydrates and alcoholates thereof.

In the further definition of symbols in the formulas hereof and where they appear elsewhere throughout this specification and in the claims, the terms have the following significance.

The term "loweralkyl" as used herein, unless otherwise specified, includes straight and branched chain radicals of up to eight carbons inclusive and is exemplified by such groups as methyl, ethyl, propyl, isopropyl, butyl, sec-butyl, tert-butyl, amyl, isoamyl, hexyl, heptyl, and octyl radicals and the like. The term "loweralkoxy" has the formula -O-loweralkyl.

The term "cycloalkyl" as used herein includes primarily cyclic alkyl radicals containing 3-7 carbon atoms inclusive and includes such groups as cyclopropyl, cyclobutyl, cyclopentyl, cyclohexyl, cycloheptyl and methylcyclohexyl and the like.

The heterocyclic amines useful in the antiallergy use of this invention are disubstituted and have the general formula:

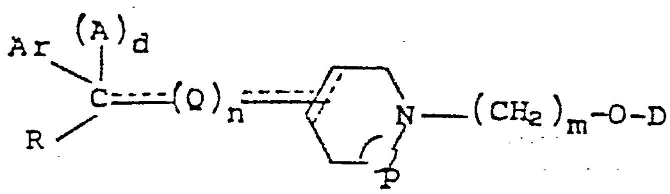

Formula I

wherein;

p is zero, one or two;

m is one to six inclusive;

A is hydrogen, hydroxy, or cyano;

d is zero or one;

Q is -CH-, $-CH_2-$ or 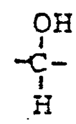

n is zero or one;

and when Q is -CH- and n is 1, a double bond is formed with one of the adjacent carbons, but not both, and when n and d are zero at the same time, a double bond is formed between the $\alpha$-carbon and a carbon of the central heterocyclic amine ring;

Ar, D and R are selected independently from the group:

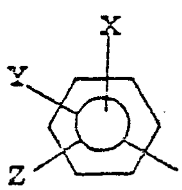 , 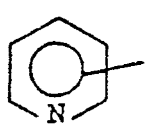 , 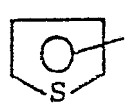 , 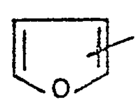 or 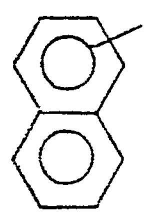

and in addition, R may have the values:

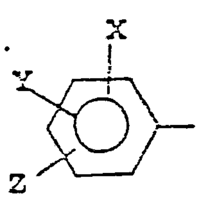 $CH_2$-, cycloalkyl or loweralkyl; and

D may have additionally the values:

The term "halo" or "halogen" when referred to herein includes fluorine, chlorine, bromine and iodine unless otherwise stated.

The term "central heterocyclic amine ring" refers to that portion of Formula I represented by 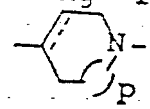

The term "phenylloweralkyl" includes phenyl connected by hydrocarbon chains exemplified by loweralkyl above and wherein phenyl may be substituted by non-reactive or non-interfering radicals such as halo, loweralkyl, loweralkoxy, and the like.

"Pharmaceutically acceptable salts" include acid addition salts, hydrates, alcoholates and quaternary salts of the compounds of Formula I which are physiologically compatible in warm-blooded animals. The acid addition salts may be formed by either strong or weak acids. Representative of strong acids are hydrochloric, hydrobromic, sulphuric, and phosphoric acids. Representative of weak acids are fumaric, maleic, mandelic, tartaric, citric, oxalic, succinic, hexamic, and the like. Suitable quaternary salts include the loweralkyl halides and loweralkyl sulphates.

The primary screening method used to detect anti-allergy properties of the compounds of Formula I is a modification of the procedure of R. R. Martel and J. Klicius, Intern. Arch. Allergy Appl. Immunology, vol. 54, pp 205-209 (1977) which measures the effect of oral administration of the compound on the volume of a rat paw which was previously injected with anti-egg albumin serum and is described in detail under Pharmacology Methods hereinbelow.

A method of studying potency in preventing guinea pig anaphylaxis relative to known anti-allergy drugs is also described hereinbelow under Pharmacology Methods. Generally, about 200 times more theophylline and 5-15 times more oxatomide are required than the more active compounds used in the present method.

Preferred compounds for use in the invention include any or all of the following:

those wherein Ar is unsubstituted phenyl or 4-substituted phenyl;

those wherein Ar is halo-substituted phenyl, trifluoromethyl-substituted phenyl, loweralkyl-substituted phenyl or lower-alkoxy substituted phenyl;

those wherein R is phenyl, 4-substituted phenyl or loweralkyl;

those wherein R is halo-substituted phenyl, loweralkyl-substituted phenyl or loweralkoxy-substituted phenyl;

those wherein M is two to five inclusive;

those wherein p is one; and

those wherein the left hand substituent in the formula, as drawn, is in the 4-position relative to the ring nitrogen atom.

DETAILED DESCRIPTION OF THE INVENTION

Anti-allergy agents of Formula I above useful in the method of treating allergy of this invention may be prepared by methods described in U. S. Patents 3,922,276 and 4,032,642. One of the general methods used in the detailed examples hereinbelow is outlined by equation in Chart I (Method A). This reaction can be carried out in alcoholic solvents, preferably refluxing butanol or in dimethylformamide, dimethoxyethane in the presence of an acid receptor as, for example, an alkali-metal carbonate, and preferably using potassium iodide catalyst. The reaction time may vary from a few hours to 24 hr, depending on reactivity of the aryloxyalkyl halide and temperature. Temperature can vary from about 80°C. to 125°C. Products are isolated, usually by partitioning in a solvent such as methylene chloride, chloroform or benzene and the like and a weak basic aqueous solution and washing, drying and concentrating the organic layer to give the free base which may then be converted, if desired, to an acid addition salt in a conventional manner.

Alternate Method B is shown by equation in Chart II. This reaction may be carried out in a suitable solvent such as tetrahydrofuran at room temperature for several hours. Preparation and isolation of the free base and a salt is typically described in Example 4.

Alternate Method C is shown by equation in Chart III. This reaction is suitable only when there is no other hydroxy radical present.

Mesylation or tosylation with such as mesyl or tosyl chloride is conducted in the presence of an acid receptor such as a tertiary amine; e.g., triethylamine, while cooling. The final reaction of the mesylate or tosylate with the $D\text{-}OM^+$ is conducted in a suitable organic solvent and the product free base is isolated by conventional means such as washing, extracting with an acid solution and an organic solvent and evaporating the solvent.

Alternate Method D is shown by equation in Chart IV. The method is limited to preparation of certain derivatives

such as wherein D is 2-pyridinyl or 2-quinolinyl. Dimethyl sulfoxide is a suitable solvent and 60°C. is a suitable temperature for the reaction.

CHART I

Preparation of Compounds of Formula I:

Method A.

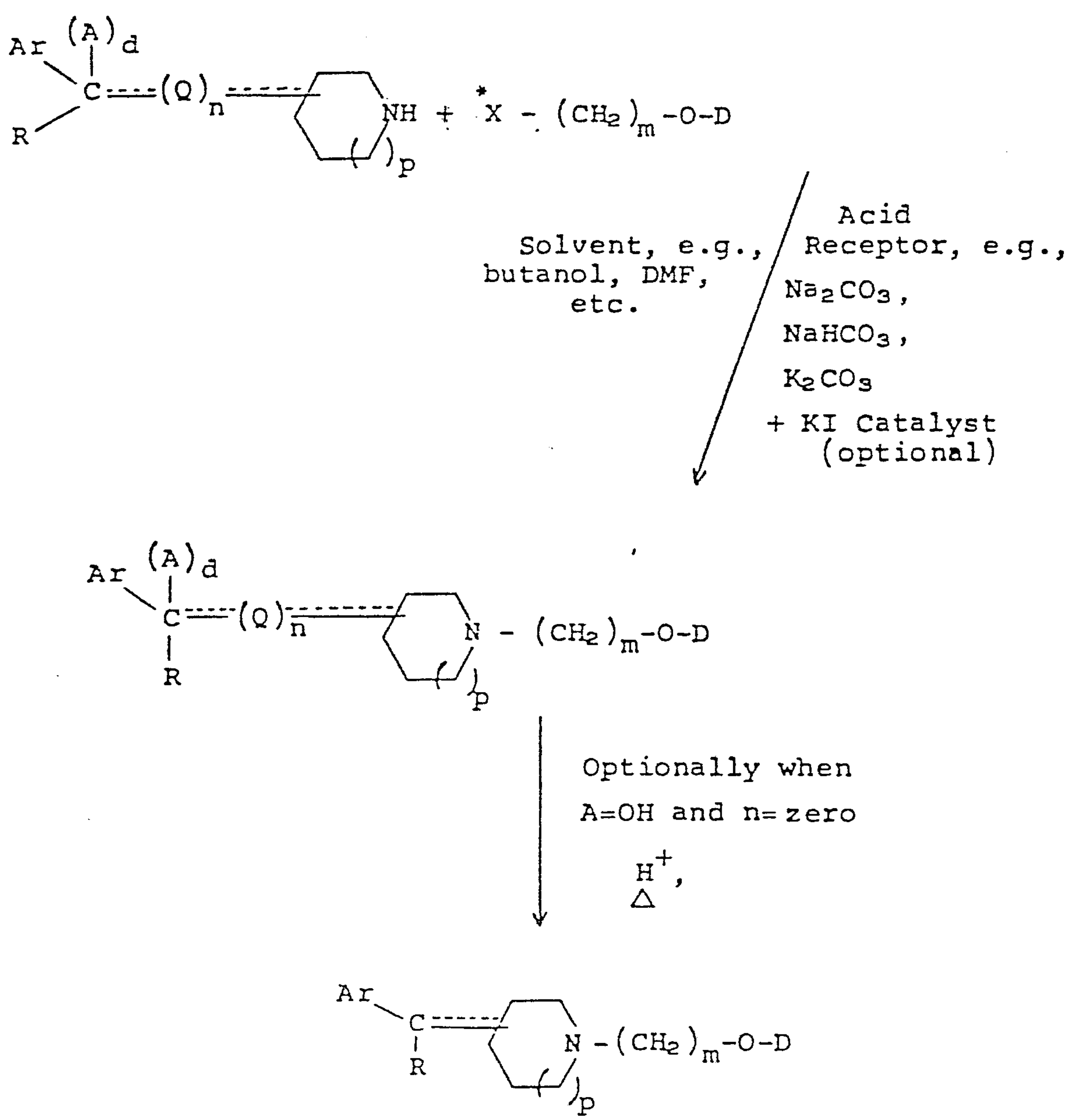


*X = halide

## CHART II

Alternate Preparation of Compounds of Formula I:

Method B.

(R = Ar, Q = zero)

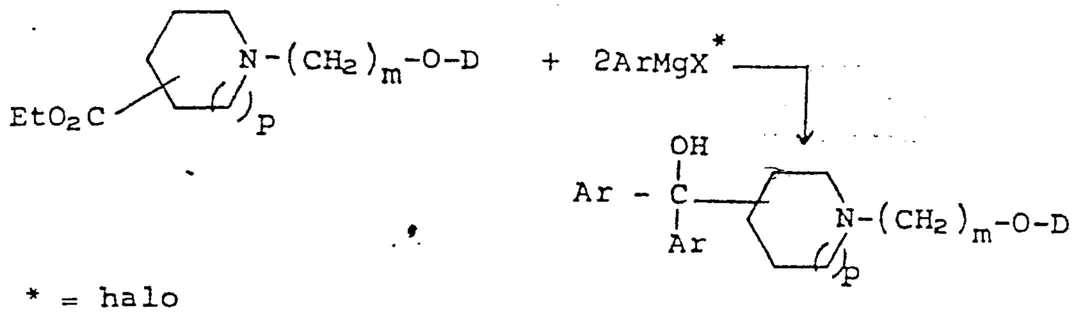


* = halo

## CHART III

Alternate Preparation of Compounds of Formula I:

Method C. When No Other Hydroxy is Present.

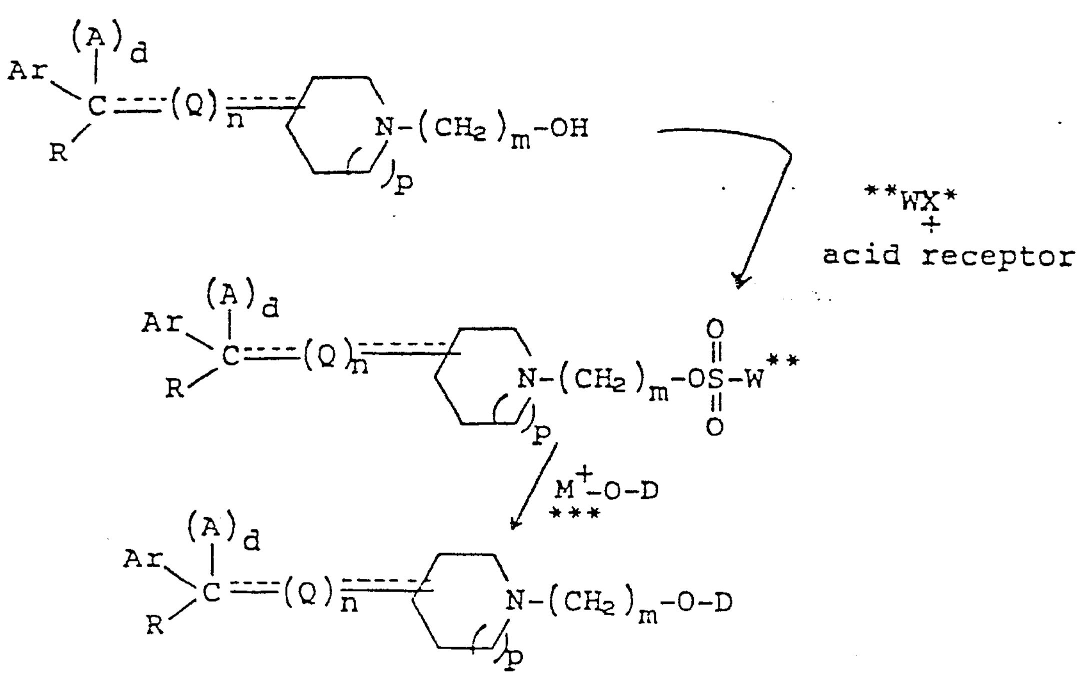


Footnotes:

*X = halo.

**W = mesyl, tosyl, etc.

***M = alkali-metal ion.

CHART IV

Alternate Preparation of Compounds of Formula I:

Method D.

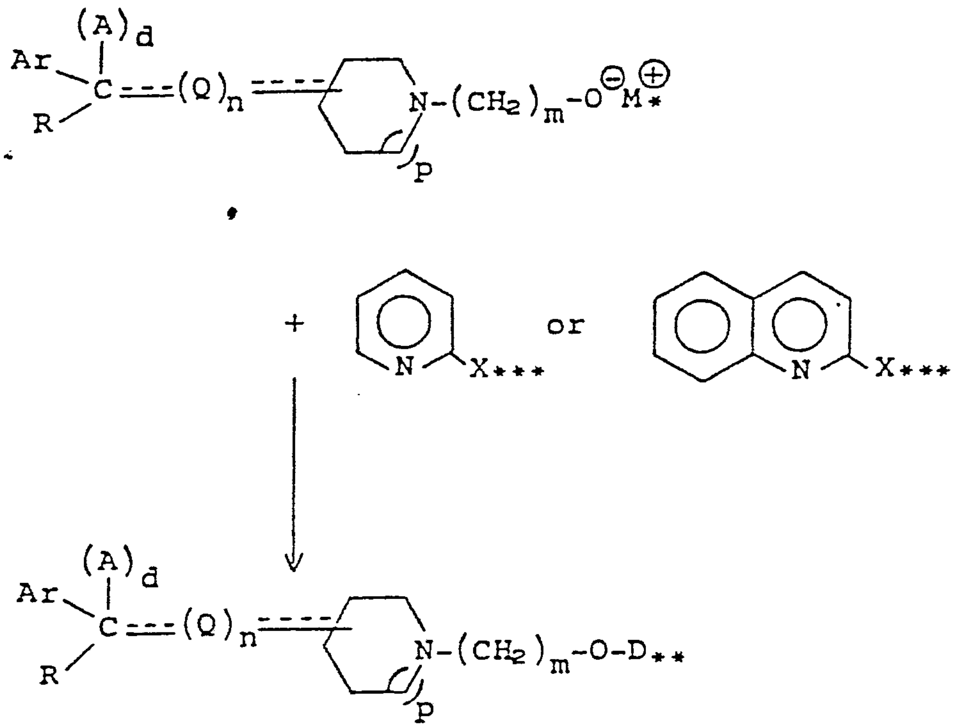

*M⊕ = alkali-metal ion.
**D = pyridin-2-yl or quinolin-2-yl.
***X = halo (Br, Cl).

To prepare acid addition salt, the free base is reacted with the calculated amount of organic or inorganic acid in aqueous miscible solvent such as ethanol or isopropanol, with isolation by concentration and/or cooling, or the base is reacted with an excess of the acid in aqueous immiscible solvent such as diethyl ether or isopropyl ether, with the desired salt separating directly. Exemplary of such organic salts are those formed with oxalic, maleic, fumaric, benzoic, ascorbic, pamoic, succinic, methanesulfonic, acetic, propionic, tartaric, citric, lactic, malic, citraconic, itaconic, hexamic, p-aminobenzoic, glutamic and stearic acid and the like. Exemplary of such inorganic salts are those formed with hydrochloric, hydrobromic, sulfuric, sulfamic, phosphoric and nitric acids.

If desired, the free base may be regenerated by proportioning the acid addition salt between an organic solvent such as methylene chloride and a weakly basic aqueous solution of, for example, sodium bicarbonate and separating the methylene chloride layer and evaporating it.

While, as stated above, the compounds of Formula I have generally exhibited positive anti-allergy utility, certain compounds encompassed by Formula I are more potent and therefore preferred and have the formula:

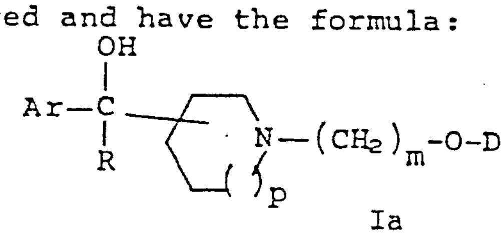

Ia

wherein p is zero or one; Ar, R, m and D have the values assigned under Formula I, and the pharmaceutically acceptable acid salts thereof.

Precursors (Chemical Intermediates) used in the synthesis of compounds of Formula I are prepared in a number of ways as illustrated by the following (1) to (9) sets of equations which are also applicable to pyrrolidinyl and homopiperidinyl derivatives. (See also U. S. Patents 3,922,276 and 3,956,296):

(1)

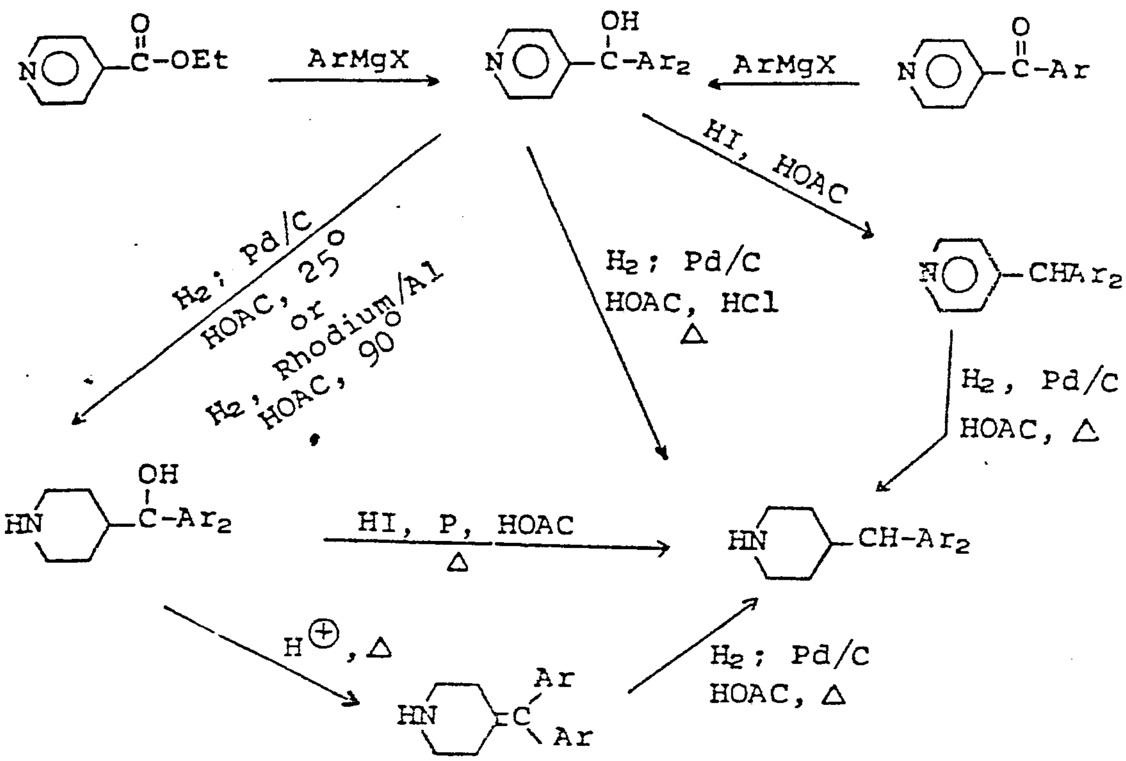
ArMgX
ArMgX
HI, HOAC
$H_2$; Pd/C
HOAC, 25°
or
$H_2$, Rhodium/Al
HOAC, 90°
$H_2$; Pd/C
HOAC, HCl
Δ
$H_2$, Pd/C
HOAC, Δ
HI, P, HOAC
Δ
$H^{\oplus}$, Δ
$H_2$; Pd/C
HOAC, Δ

(2)

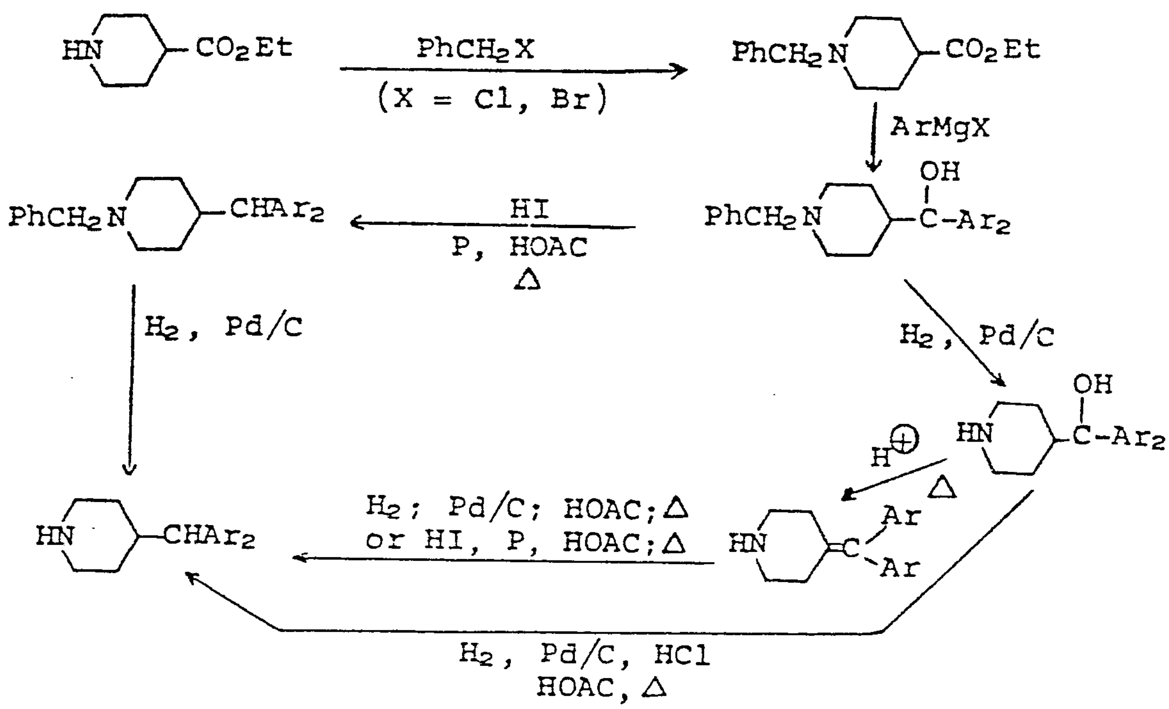
$PhCH_2X$
(X = Cl, Br)
ArMgX
HI
P, HOAC
Δ
$H_2$, Pd/C
$H_2$, Pd/C
$H^{\oplus}$
Δ
$H_2$; Pd/C; HOAC; Δ
or HI, P, HOAC; Δ
$H_2$, Pd/C, HCl
HOAC, Δ

Ph = phenyl.

(3)

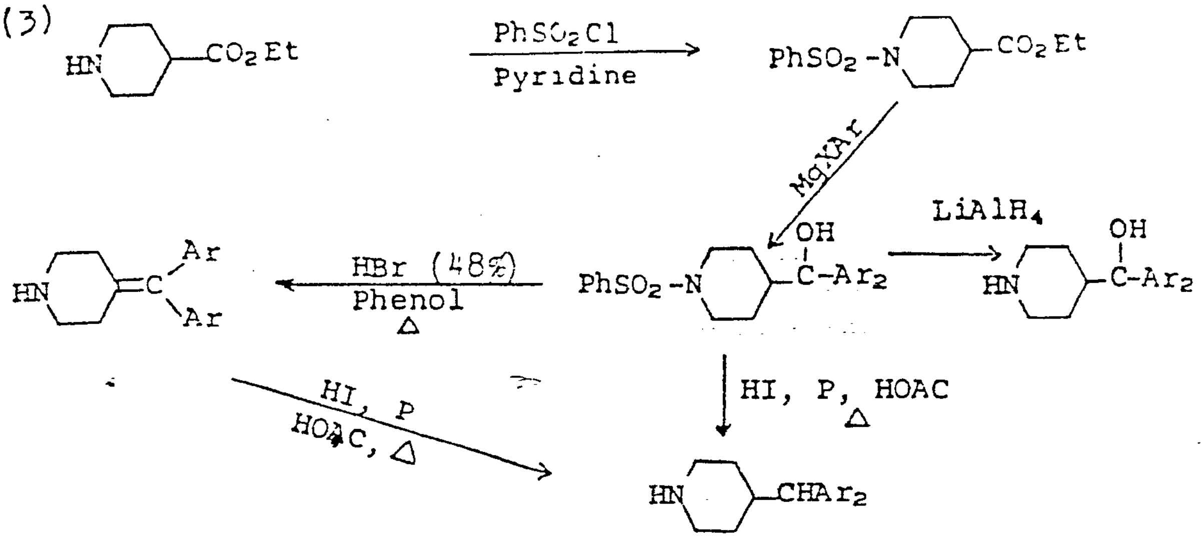


(4)

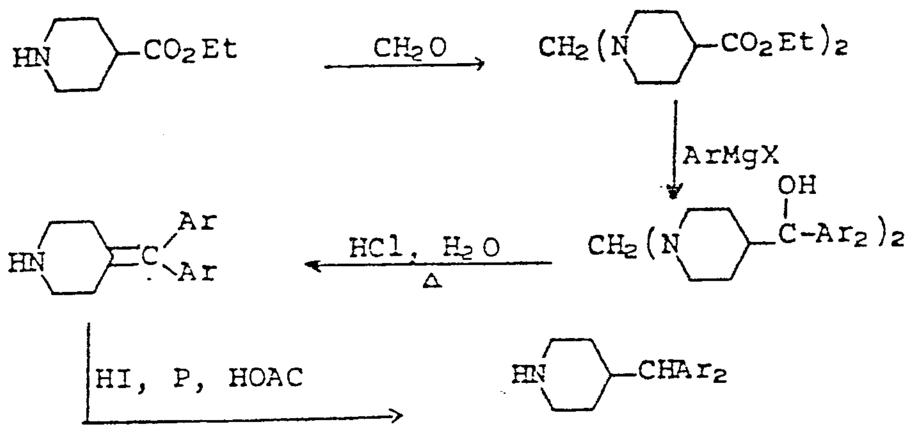


(5)

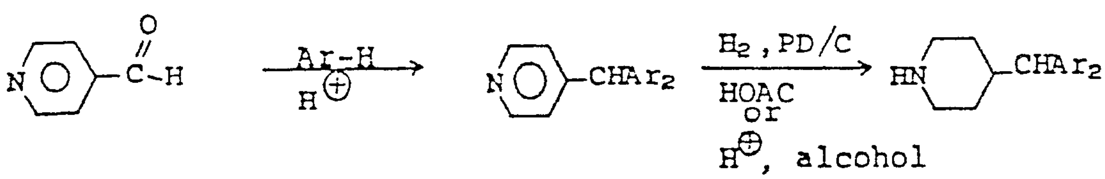


(6)

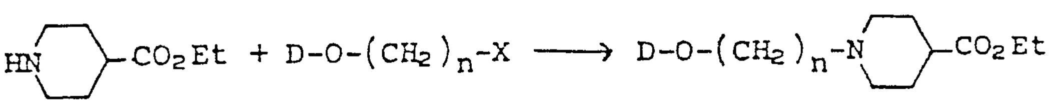

X = Cl, Br

Ph = phenyl.

(7)

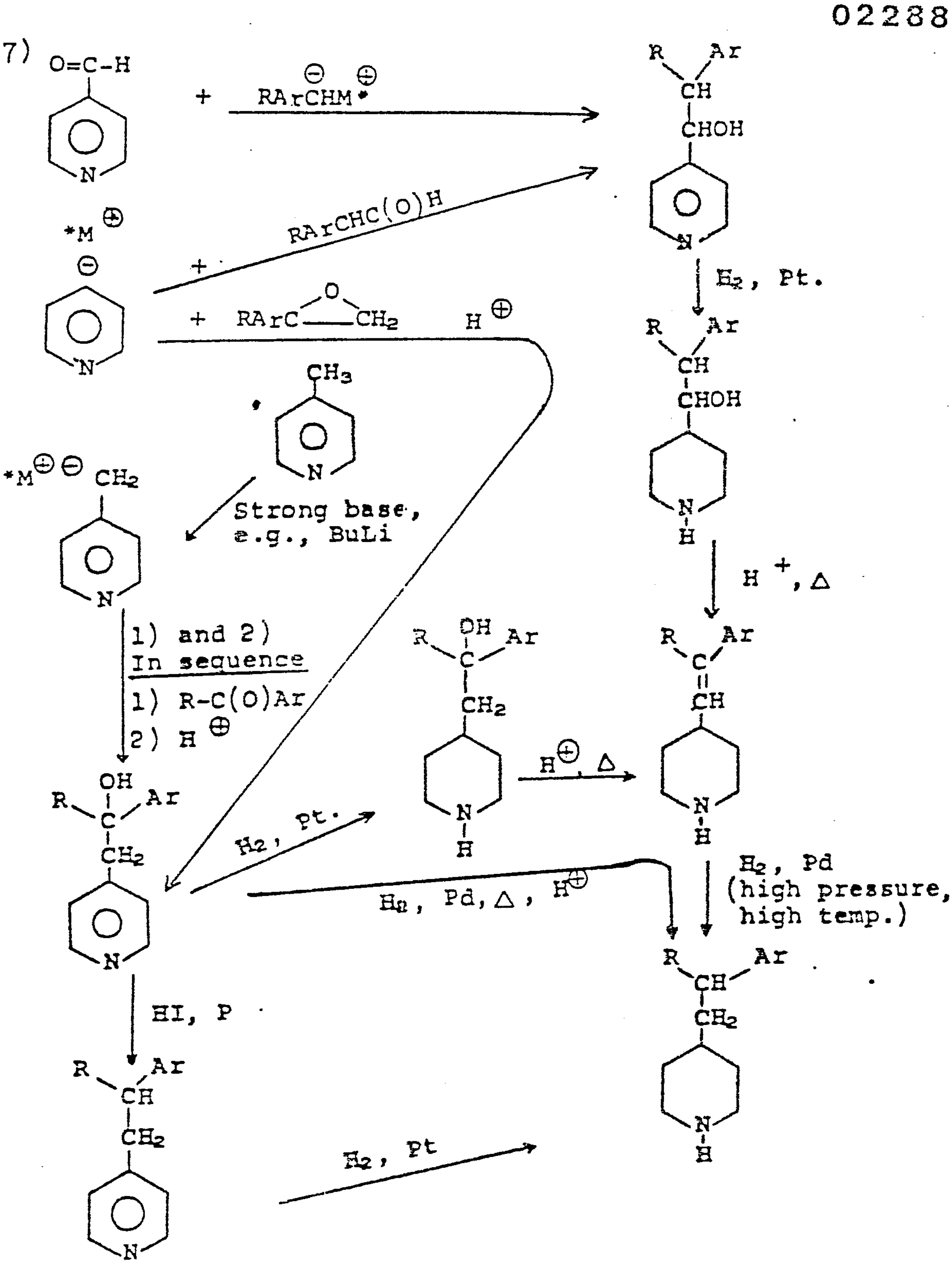


$*M^{\oplus}$ = $Li^{\oplus}$ or $MgBr^{\oplus}$

(8)

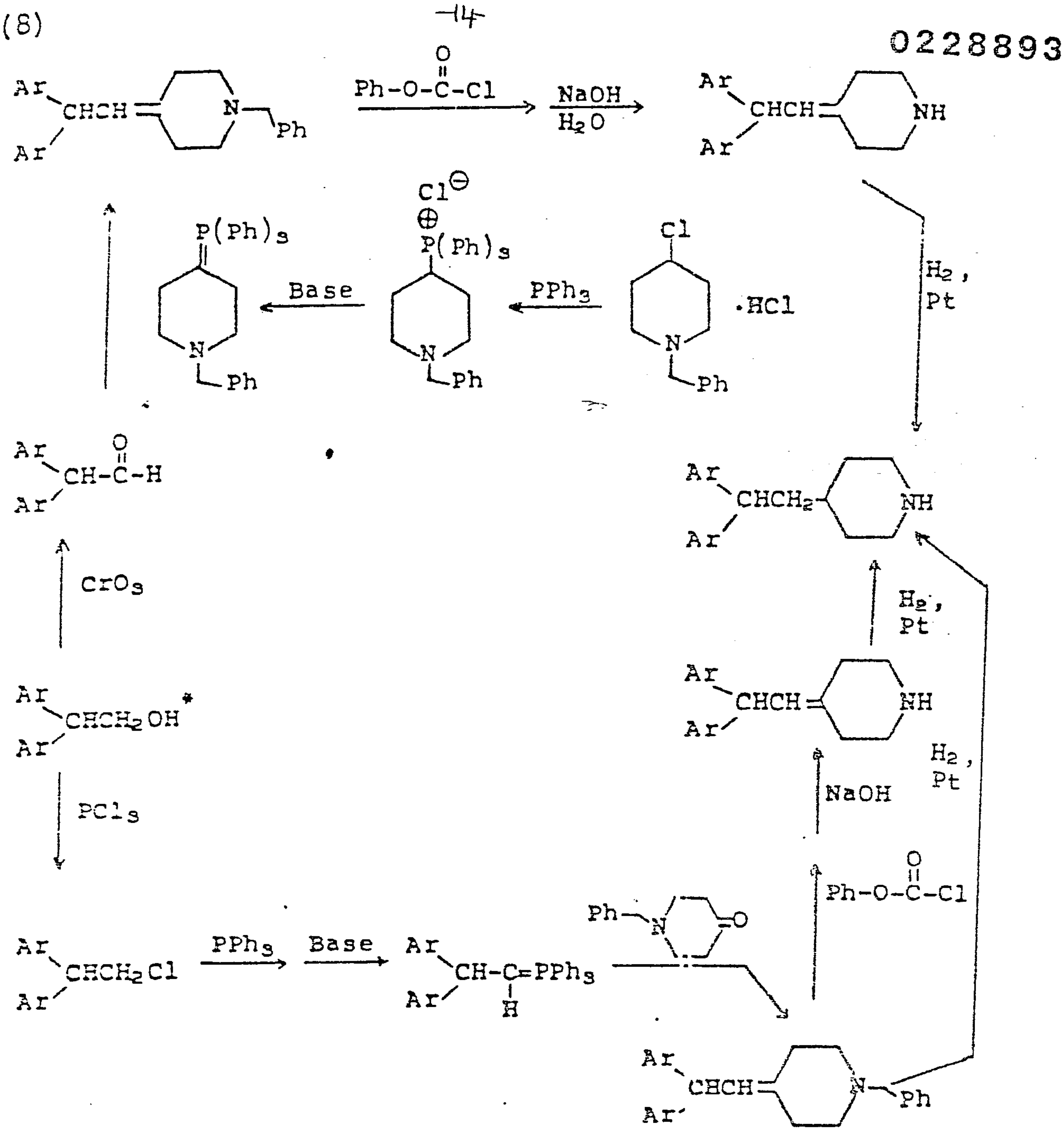


*Commercially available
Ph = phenyl

(9)

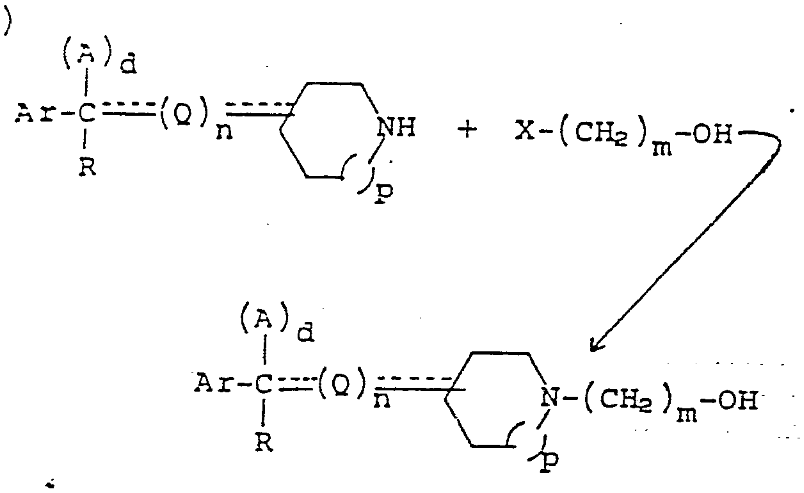

X = halo.

The method of preparation of certain starting materials wherein D is phenyl substituted by hydroxy is illustrated by the following equations:

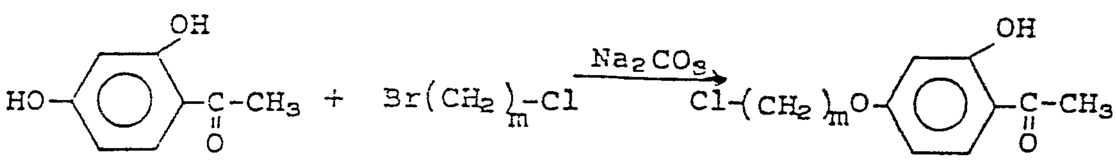


The preparation of other hydroxyphenyl intermediates and compounds is illustrated by the following equation:

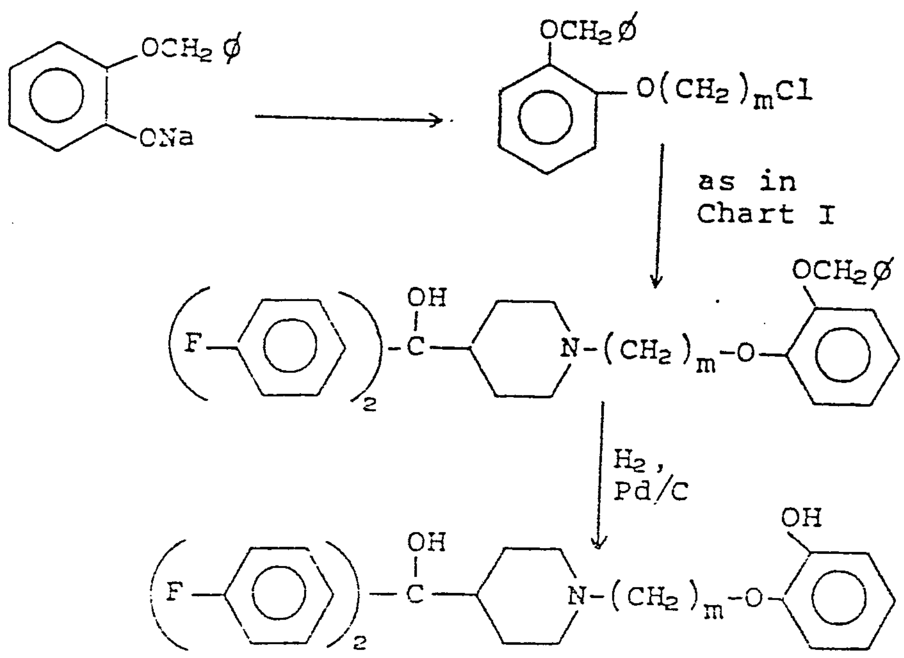


ø = phenyl.

The preparation of certain substituted phenol starting materials is illustrated by the following equations:

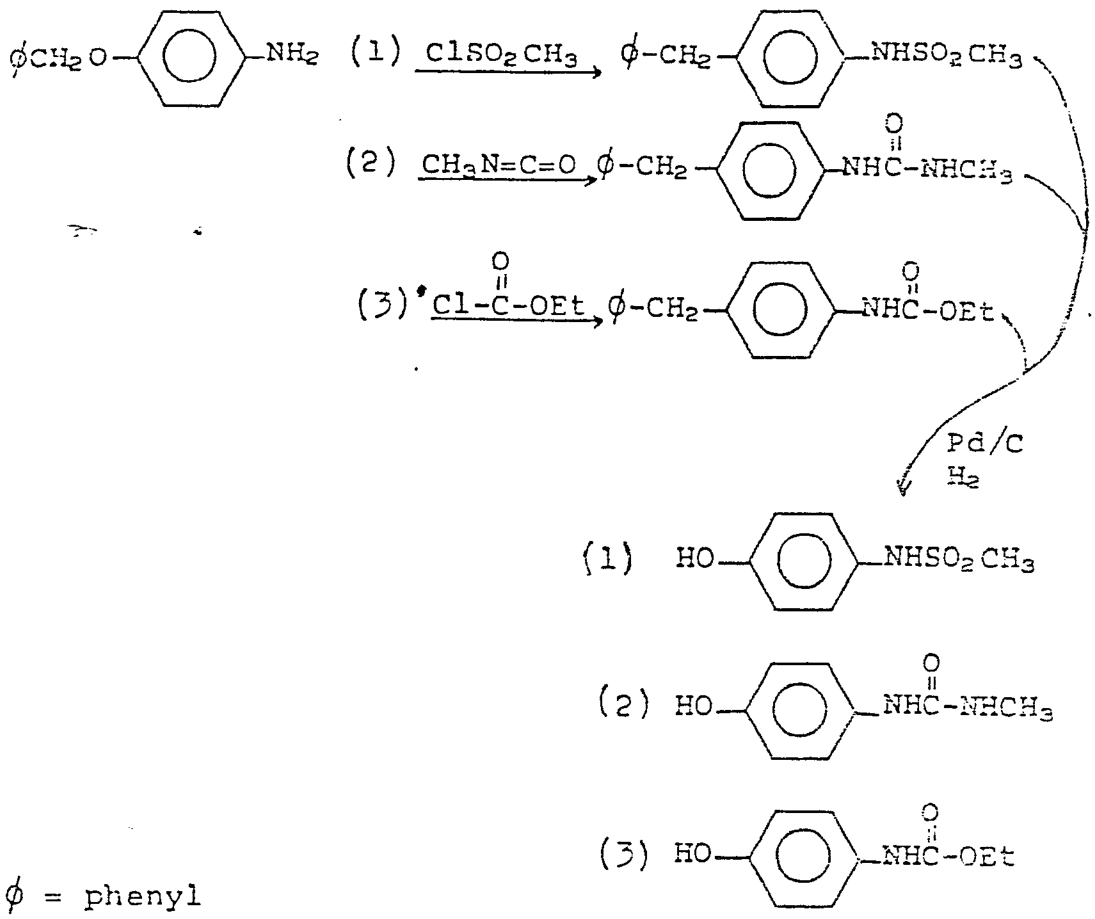


ϕ = phenyl

The preparation of chemical intermediates is further illustrated in the following Preparations 1 to 64. Examples 1 to 114 illustrate the synthesis methods for preparing compounds of Formula I. The scope of the invention is not limited by the descriptive methods and procedures of the preparations and examples, however.

Preparation 1

4-Diphenylmethylenepiperidine.

A solution of 7.0 g of 1-acetyl-4-diphenylhydroxymethylpiperidine in 30 ml of absolute alcohol and 76 ml of concentrated hydrochloric acid was heated at reflux for seven hours, cooled and made basic with 50% sodium hydroxide. The oil which separated was extracted with benzene and the combined extracts washed with water. After drying over magnesium sulfate the solvent was evaporated at reduced pressure. The residual oil which crystallized on cooling was recrystallized twice from petroleum ether to give 4.0 g (73.0%) of white crystals, m.p. 85-86°C.

Analysis: Calculated for $C_{18}H_{19}N$: C,86.70; H,7.68; N,5.62
Found : C,85.70; H,7.83; N,5.73

Preparation 2

[α,α-Bis(p-fluorophenyl)]-4-piperidinemethanol hydrochloride hydrate [1:1:0.5].

This compound was prepared by the method described in Preparation 1 of U. S. Patent 4,032,642, m.p. 243-243.5°C. from the Grignard reagent formed with p-fluorobromobenzene and 1-acetyl-4-(p-fluorobenzoyl)piperidine followed by hydrolysis and conversion to the salt.

Preparation 3

1-(Phenylmethyl)-4-piperidinecarboxylic acid ethyl ester hydrochloride [1:1].

A mixture of 100 g (0.637 mole) of ethyl isonipecotate, 80.64 g (0.64 mole) of benzyl chloride and 67.84 g (0.64 mole) of sodium carbonate in 1 liter of absolute ethanol was refluxed for 8 hours and then was stirred at room temperature for 10 hours. The solvent was removed *in vacuo*, and the residue was partitioned between methylene chloride and dilute sodium hydroxide. The methylene chloride phase was dried over magnesium sulfate and the solvent was removed *in vacuo* to give the free base of the title compound as a liquid. The free base was converted to the hydrochloric acid salt, and the salt was recrystallized from ethanol-ether to give 89.33 g (49.7%) of white crystalline solid, m.p. 154-155°C.

Analysis: Calculated for $C_{15}H_{22}NO_2Cl$: C,63.48; H,7.81; N,4.94
Found : C,63.07; H,7.82; N,4.91

Preparation 4

α,α-Bis-(4-fluorophenyl)-1-(phenylmethyl)-4-piperidine-methanol.

To a 6.08 g (0.25 mole) of magnesium turnings and an iodine crystal in 600 ml of dry tetrahydrofuran and under an atmosphere of nitrogen was added, dropwise, a solution of p-bromofluorobenzene in 125 ml of tetrahydrofuran. The temperature of the reaction was kept below 10°C. by cooling in an ice-methanol bath. The mixture was stirred at room temperature for 1.5 hours. A solution of 24.7 g (0.10 mole) of 1-(phenylmethyl)-4-piperidinecarboxylic acid ethyl ester hydrochloride in tetrahydrofuran was added, and the mixture was stirred at room temperature for 17 hours. The reaction was poured into an icy, aqueous solution of ammonium chloride, and the resulting solution was extracted with methylene chloride. The methylene chloride solution was extracted with dilute sodium hydroxide and was dried over magnesium sulfate. The solvent was removed in vacuo to give an oil. This was crystallized from ether-hexane to give 19.87 g (51%) of the title compound, m.p. 113-115°C.

Analysis: Calculated for $C_{25}H_{25}NOF_2$: C,76.31; H,6.40; N,3.56
Found : C,76.24; H,6.38; N,3.50

Preparation 5

[α,α-Bis(p-fluorophenyl)]-4-piperidinemethanol.

A solution of 31.2 g (0.079 mole) of α,α-bis-(4-fluorophenyl)-1-(phenylmethyl)-4-piperidinemethanol in 400 ml of absolute ethanol was hydrogenated at 50 psi and 70°C. over 5% palladium on carbon over the weekend. The mixture was filtered and the filtrate was concentrated under reduced pressure to give a gum as residue. Methylene chloride was added to the residue and the gum crystallized. The mixture was diluted with petroleum ether and the solid was collected by filtration, washed with petroleum ether, and dried to yield 22 g (92%) of white solid which was recrystallized from isopropyl ether-2-propanol, m.p. 159.5-160.5°C.

Analysis: Calculated for $C_{18}H_{19}F_2NO$: C,71.27; H,6.31; N,4.62
Found : C,70.93; H,6.71; N,4.38

Preparation 6

1-(Phenylsulfonyl)-4-piperidinecarboxylic acid, ethyl ester.

To a solution of 10.1 g (0.0642 mole) of ethyl isonipecotate in 300 ml of pyridine and cooled in an ice bath was added 13.2 g (0.075 mole) of benzene sulfonyl chloride. The mixture was stirred for 2 hours at room temperature, and the solvent was removed *in vacuo*. The residue was partitioned between methylene chloride and dilute sodium hydroxide. The methylene chloride solution was dried over magnesium sulfate, and the solvent was removed *in vacuo* to give a solid. This was recrystallized from ethanol-ether to give 4.59 g (24.1%) of crystalline solid; m.p. 85-86.5.

Analysis: Calculated for $C_{14}H_{19}NO_4S$: C,56.55; H,6.44; N,4.71
Found : C,56.53; H,6.55; N,4.67

In another preparation, 100 g (0.634 mole) of ethyl nipecotate and 130.4 g (0.74 mole) of benzene sulfonyl chloride were reacted by the above procedure for 4-1/2 hr. to give the title product in 78.1% yield.

Preparation 7

α,α-Bis(4-fluorophenyl)-1-(phenylsulfonyl)-4-piperidinemethanol.

To a suspension of 33.78 g (1.39 mole) of magnesium trimmings in 1 liter of tetrahydrofuran (dried over molecular sieves 5A) under an atmosphere of $N_2$ and cooled in an ice bath was added dropwise a solution of 243.25 g (1.39 mole) of p-bromofluorobenzene in 150 ml of tetrahydrofuran. The mixture was stirred for 2 hr after the addition was completed. To this mixture was added 103 g (0.346 mole) of 1-(phenylsulfonyl)-4-piperidinecarboxylic acid ethyl ester as a solid, and the solution was stirred at ambient temperature for 5 hr. The reaction was poured into an icy aqueous solution of ammonium chloride. The phases were separated, and the solvent was removed *in vacuo* from the organic phase. The

BAD ORIGINAL